(19) Europäisches Patentamt
European Patent Office
Office européen des brevets

(11) EP 2 597 086 A1

(12) EUROPEAN PATENT APPLICATION
published in accordance with Art. 153(4) EPC

(43) Date of publication:
**29.05.2013 Bulletin 2013/22**

(21) Application number: **11809933.2**

(22) Date of filing: **01.07.2011**

(51) Int Cl.:
***C07D 239/10*** *(2006.01)* ***C07D 239/22*** *(2006.01)*
***C07D 233/86*** *(2006.01)* ***C07D 233/72*** *(2006.01)*
***C07D 471/10*** *(2006.01)* ***C07D 491/107*** *(2006.01)*
***C07D 249/12*** *(2006.01)* ***A61K 31/4196*** *(2006.01)*
***A61K 31/513*** *(2006.01)* ***A61K 31/4166*** *(2006.01)*
***A61K 31/4188*** *(2006.01)* ***A61K 31/527*** *(2006.01)*
***A61K 9/20*** *(2006.01)* ***A61K 9/48*** *(2006.01)*
***A61K 9/08*** *(2006.01)* ***A61P 35/00*** *(2006.01)*

(86) International application number:
**PCT/RU2011/000476**

(87) International publication number:
**WO 2012/011840 (26.01.2012 Gazette 2012/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.07.2010 RU 2010130618**

(71) Applicants:
- **Ivachtchenko, Alexandre Vasilievich**
  **Encinitas, CA 92024 (RU)**
- **Ivashchenko, Andrey Alexandrovich**
  **Moscow 127576 (RU)**
- **Savchuk, Nikolay Filippovich**
  **Moscow 117420 (RU)**

(72) Inventor: **MITKIN, Oleg Dmitrievich**
**Moskovskaya obl. 141400 (RU)**

(74) Representative: **Patentanwälte**
**Zellentin & Partner**
**Rubensstrasse 30**
**67061 Ludwigshafen (DE)**

(54) **CYCLIC N,N'-DIARYLTHIOUREAS AND N,N'-DIARYLUREAS AS ANDROGEN RECEPTOR ANTAGONISTS, ANTI-CANCER AGENT, METHOD FOR PRODUCING AND USING SAME**

(57) The present invention relates to novel cyclic N, N'-diarylureas and N,N'-diarylthioureas - androgen receptor antagonists, anti-cancer agent, pharmaceutical composition, medicament, and method for treatment of cancerous diseases, among them prostate cancer.

Cyclic *N,N'*-diarylthioureas or *N,N'*-diarylureas of the general formula 1, their optical (R)- and (S)- isomers and pharmaceutically acceptable salts thereof exhibiting properties of androgen receptor antagonists have been proposed,

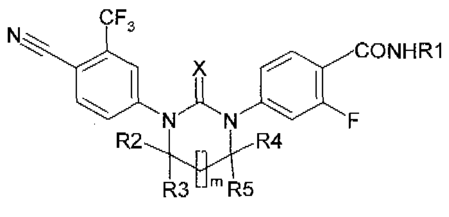

**1**

wherein: **X** represents oxygen or sulfur;
**m** = 0 or 1;
**R1** represents $C_1$-$C_3$alkyl;
**R2** and **R3** represent hydrogen; or
**R2** and **R3** together with C-atom they are attached to form C = O group; **R4** and **R5** represent hydrogen; or
**R4** represents hydrogen, **R5** represents methyl; or
**R4** represents methyl, **R5** represents **$CH_2$R6** group in which **R6** represents $C_1$-$C_3$ alkoxycarbonyl, carboxyl, hydroxyl group optionally substituted with methyl or benzyl; or
**R4** and **R5** together with C-atom they are attached to form 5- or 6- membered heterocycle comprising at least one oxygen atom or nitrogen atom optionally substitutd with methyl; or
**R4** and **R5** together with C-atom they are attached to form NH group.

EP 2 597 086 A1

## Description

### Field of the invention

The invention relates to novel cyclic N,N'-diarylthioureas and N,N'-diarylureas-androgen receptor antagonists, anticancer agent, pharmaceutical composition, medicament and method for treatment of cancer including prostate cancer.

### Prior art

There are known androgen receptor antagonists which are -1,3-diaryl-5,5-dimethyl-2-thioxoimidazolidin-4-ones I, 5,7-diaryl-6-thioxo-5,7-diazaspiro[3,4]octan-8-ones **II** and 1,3-diaryl-2-thioxo-1,3-diazaspiro[4,4]nonan-4-ones **III** exhibiting anticancer activity [WO2006124118, WO2007127010]. Amongst these compounds the most promoted is 4-[3-[4-cyano-3-(trifluoromethyl)phenyl]-5,5-dimethyl-4-oxo-2-thioxoimidazolidin-1-yl]-2-fluoro-N-methylbenzamide **MDV3100** (androgen receptor antagonist with $IC_{50}$= 36 nM), which is now in the III phase of clinical trials as medicament for prostate cancer treatment [Drug Data Rep., 2009, 31(6), 609].

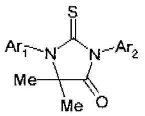
I

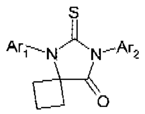
II

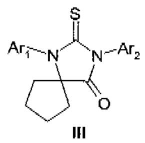
III

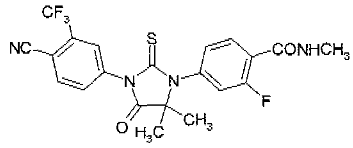
MDV3100

Searching for highly effective anticancer medicaments exhibiting enhanced activity and reduced toxicity, is still one of the main directions in the development of novel pharmacological remedies for cancer treatment, including prostate cancer. In this context the development of novel anticancer active agents, pharmaceutical compositions and medicaments, and also methods for their preparation and use is of essential importance.

### Disclosure of the invention

In the context of the invention, the terms are generally defined as follows:

**"Azaheterocycle"** means an aromatic or non aromatic mono- or poly- cyclic system, comprising at least one nitrogen atom in the cycle. Azaheterocycle may have one or more "cyclic system" substituents.

**"Active component"** (drug-substance) means a physiologically active compound of synthetic or other origin (biotechnological, vegetable, animal, microbe and so on), exhibiting pharmacological activity and being an active component of pharmaceutical composition, employing in production and preparation of medicaments.

**"Alkyl"** means an aliphatic hydrocarbon straight or branched chain with 1-12 carbon atoms. Branched means an alkyl chain with one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy and so on.

**"Antagonists"** mean ligands which being bound to definite receptors not cause active cellular responses. Antagonists prevent linkage between agonists and receptors and by that block specific receptor signal transmission.

**"Aryl"** means aromatic mono- or poly- cyclic system with 6 - 14 carbon atoms, predominantly 6-10 carbon atoms. Aryl may have one or more "cyclic system substituents" of the same or different structure. Phenyl, substituted phenyl, naphthyl, or substituted naphthyl are the representatives of aryl groups. Aryl could be annelated with nonaromatic cyclic system or heterocycle.

**"Heterocyclyl"** means aromatic or saturated mono- or polycyclic system with 3 - 10 carbon atoms, preferably from 5 to 6, wherein one or more carbon atoms are substituted by one or more heteroatoms, such as N, S or O. Prefix "aza", "oxa" or "thia" before "heterocyclyl" means that N, O or S atoms are introduced in the cycle, respectively. Heterocyclyl may have one or more "cyclic system sustituents" of the same or different structure. N- and S- atoms of heterocyclyl cycle could be oxidized to N-oxide, S-oxide or S-dioxide. Piperidinyl, pyrrolidinyl, piperazinyl, morpholinyl, thiomorpholinyl, thiazolidinyl, 1,4-dioxane-2-yl, tetrahydrofuranyl, tetrahydrothiophenyl and others are examples of heterocyclyl.

**"Hydrate"** means stoichiometric or nonstoichiometric compositions of compounds or their salts with water.

**"Substituent"** means a chemical radical attached to scaffold (fragment), for example, "alkyl substituent", "amino

group substituent", "carbamoyl substituent", and "cyclic system substituent", the meanings of which are defined in this section.

**"Medicament" -** is a compound (or mixture of compounds in the form of pharmaceutical composition) in the form of tablets, capsules, injections, ointments and other ready forms intended for restoration, improvement or modification of physiological functions at humans and animals, and also for treatment and prophylaxis of diseases, diagnostics, anesthesia, contraception, cosmetology and others.

**"slower alkyl"** means a straight or branched alkyl with 1-4 carbon atoms.

**"Pharmaceutical composition"** means a composition comprising a compound of general formula **1** and at least one of components selected from the group consisting of pharmaceutically acceptable and pharmacologicaly compatible fillers, solvents, diluents, auxiliary, distributing and sensing agents, delivery agents, such as preservatives, stabilizers, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the nature and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against the action of microorganisms can be provided by various antibacterial and antifungal agents, such as, for example, parabens, chlorobutanole, sorbic acid, and similar compounds. Composition may also contain isotonic agents, such as, for example, sugar, sodium chloride, and similar compounds. Prolonged effect of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and polyethylene glycol of high molecular weight. Pharmaceutical composition for peroral, sublingval, transdermal, intramuscular, intravenous, subcutaneous, local or rectal administration of active ingredient, alone or in combination with another active compound may be administered to humans and animals in standard administration form, or in mixture with traditional pharmaceutical carriers. Suitable standard administration forms include peroral forms such as tablets, gelatin capsules, pills, powders, granules, chewing-gums and peroral solutions or suspensions, sublingval and transbuccal administration forms; aerosols; implants; local, transdermal, subcutaneous, intramuscular, intravenous, intranasal or intraocular forms of introduction and rectal administration forms. Pharmaceutical compositions are usually prepared by means of standard procedures by mixing an active compound with liquid or overgrounded solid carrier.

**"Pharmaceutically acceptable salt"** means a relatively nontoxic both organic and inorganic salts of acids and bases disclosed in this invention. Salts could be prepared in situ in processes of synthesis, isolation or purification of compounds or be prepared specially. In particular, bases salts could be prepared starting from purified base of disclosed compound and suitable organic or mineral acid.

Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of properties of such salts is given in: Berge S.M., et al., "Pharmaceutical Salts" J.Pharm.Sci., 1977, 66: 1-19). Salts of disclosed acids may be also prepared by reaction of purified acids specifically with suitable base; moreover, metal salts and amine salts may be synthesized too. Metal salts are salts of sodium, potassium, calcium, barium, magnesium, zink, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of disclosed acid salts are amines and amino acids of sufficient basicity to produce stable salt suitable for medical purposes use (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected among main aminoacids - lysine, ornithine and agrinine. The authors have disclosed novel cyclic N,N'-diarylthioureas and N, N'-diarylureas of the general formula 1, optical (R)- and (S)- isomers and pharmaceutically acceptable salts thereof which are androgen receptor antagonists:

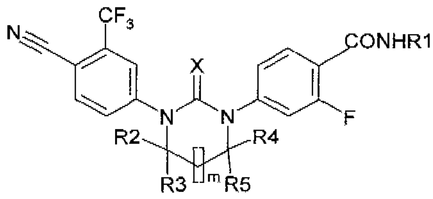

**1**

wherein:

**X** represents oxygen or sulfur;
**m =** 0 or 1;
**R1** represents $C_1$-$C_3$alkyl;
**R2** and **R3** represent hydrogen; or
**R2** and **R3** together with the C-atom they are attached to form C = O group;
**R4** and **R5** represent hydrogen; or
**R4** represents hydrogen, **R5** represents methyl; or
**R4** represents methyl, **R5** represents **$CH_2$R6** group in which **R6** is $C_1$-$C_3$ alkoxycarbonyl; carboxyl; hydroxyl group optionally substituted with methyl or benzyl; or
**R5** and **R4** together with the C-atom they are attached to form 5- or 6- membered heterocycle comprising at least one oxygen atom or nitrogen atom optionally substituted with methyl; or
**R4** and **R5** together with the C-atom they are attached to represent NH group. The preferred compounds are N,N'-diarylthioureas and N,N'-diarylureas, their optical (R)- and (S)- isomers and pharmaceutically acceptable salts of the general formulas **1.2, 1.3** or **1.4:**

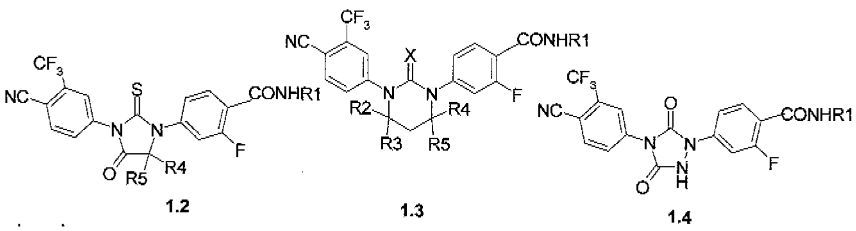

**1.2** **1.3** **1.4**

wherein:

**X, R1 , R2, R3, R4** and **R5** have the above meanings.

The more preferable compounds are cyclic N,N'-diarylthioureas of formulas **1.2(1), 1,2(2), 1.2,2** and **1.2.3,** their optical (R)-isomers - (R)**-1.2(2),** (R)-**1.2.2**, (R)-**1.2.3** and (S)-isomers - (S)-**1.2(2),** (S)-**1.2.2** and (S)-**1.2.3**:

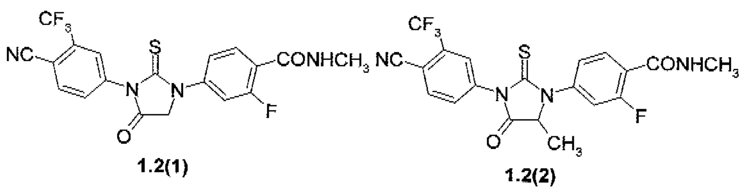

**1.2(1)** **1.2(2)**

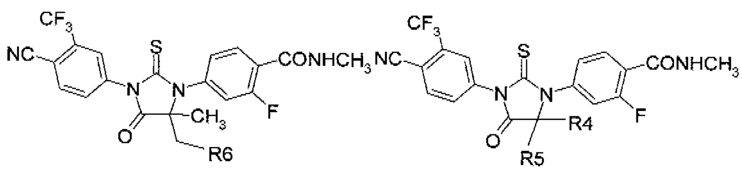

**1.2.2** **1.2.3**

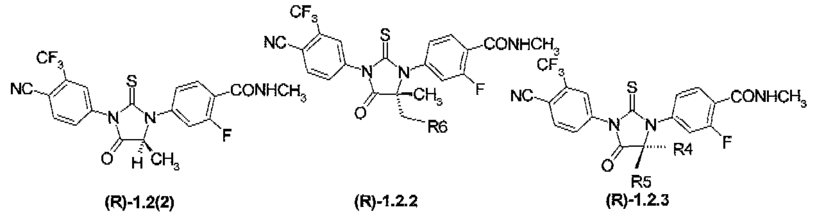

(R)-1.2(2) (R)-1.2.2 (R)-1.2.3

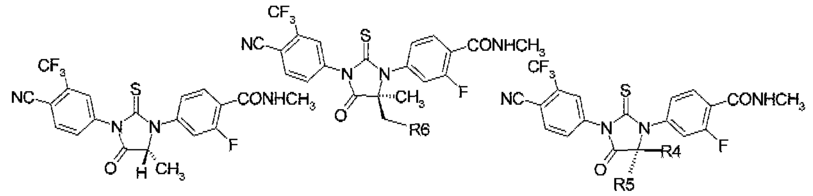

(S)-1.2(2) (S)-1.2.2 (S)-1.2.3

wherein:

**R5** and **R4** together with the C-atom they are attached to form 5- or 6- membered heterocycle comprising at least one oxygen atom or nitrogen atom optionally substituted with methyl,
**R6** has the meaning mentioned above.

The more preferable compounds are also compounds of formulas **1.2.2(1), 1.2.2(2), 1.2.2(3), 1.2.3(1), 1.2.3 (2)** and **1.2.3(3),** their optical (R)-isomers - (R)-**1.2.2(1), (R)-1.2.2(2), (R)-1.2.2(3),** (R)-**1.2.3(1),** and (S)-isomers - (S)-**1.2.2(1),** (S)-**1.2.2(2),** (S)-**1.2.2(3), (S)-1.2.3(1°),** or a pharmaceutically acceptable salt thereof,

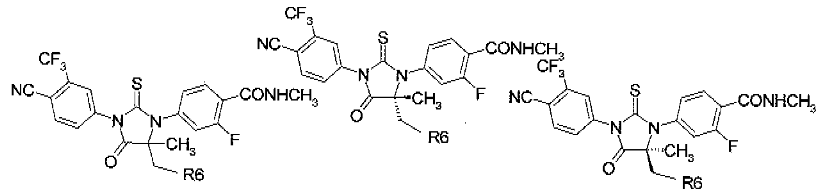

**1.2.2(1)**: R6 $=OCH_3$;
**1.2.2(2)**: R6 $=OCH_2Ph$;
**1.2.2(3)**: R6 = OH;

**(R)-1.2.2(1)**: R6 = $OCH_3$;
**(R)-1.2.2(2)**: R6 $=OCH_2Ph$;
**(R)-1.2.2(3)**: R6 = OH;

**(S)-1.2.2(1)**: R6 $=OCH_3$;
**(S)-1.2.2(2)**: R6 = $OCH_2Ph$;
**(S)-1.2.2(3)**: R6 = OH;

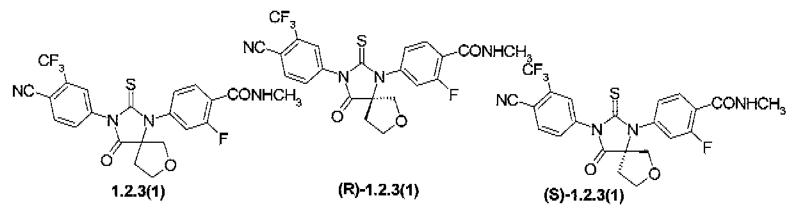

1.2.3(1) (R)-1.2.3(1) (S)-1.2.3(1)

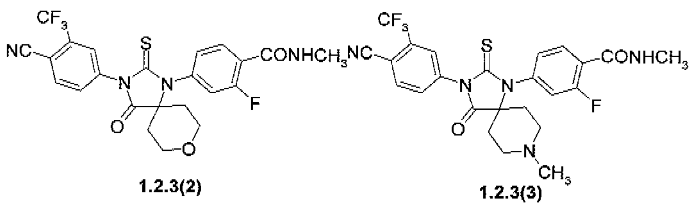

1.2.3(2) 1.2.3(3)

wherein **R6** represents hydroxyl group optionally substituted with methyl or benzyl, The subject of the present invention is a method for preparation of compounds of the general formula **1.2** and optical **(R)-** and **(S)-** isomers thereof. 1,3-Diarylhydantoines of the general formula **1.2** are prepared by interaction of isothiocyanate **3.2** with the corresponding 4-(cyanomethyl)aminobenzamides **4.1** or (4-carbamoylphenylamino) acetic acids **4.2** according to scheme 1.

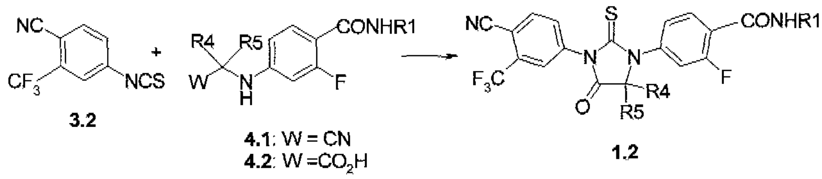

3.2

4.1: W = CN
4.2: W =$CO_2H$

1.2

wherein:

**R1,** R4 and R5 have the above meanings.

Scheme 1.

Optically active cyclic N,N'-diarylthioureas, **(R)-1.2** and **(S)-1.2** isomers, are prepared either from the corresponding optically active **(R)-4.1, (R)-4.2, (S)-4.1** and **(S)-4.2** starting materials, or by resolution of racemic mixtures of cyclic N,N'-diarylthioureas **1.2** to enantiomers.

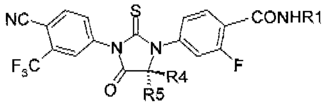

(R)-1.2

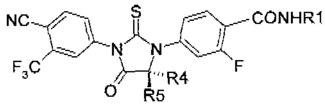

(S)-1.2

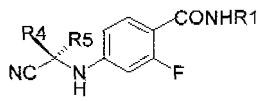

(R)-4.1

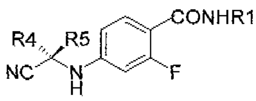

(S)-4.1

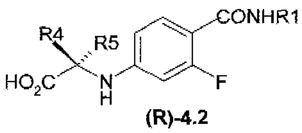

(R)-4.2

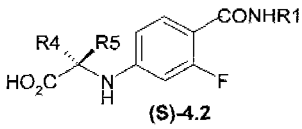

(S)-4.2

wherein:

**R1, R4** and **R5** have the above meanings.

1,3-Diaryltetrahydropyrimidin-2-ones of the general formula **1.3.1** are prepared by interaction of the corresponding *N,N'*-diarylureas of the general formula 2 with 1,3-dibromopropane according to scheme 2.

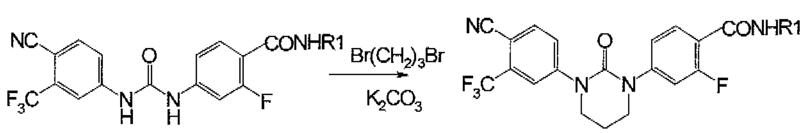


2

1.3.1

wherein:

**R1** has the above meaning.

Scheme 2.

Compounds of the general formula **1.3.2** are prepared by interaction of isocyanate **3.1** or isothiocyanate **3.2** with the corresponding ethyl β-alaninates of the general formula **5** with subsequent cyclization of obtained ureas of the general formula **6** according to scheme 3.

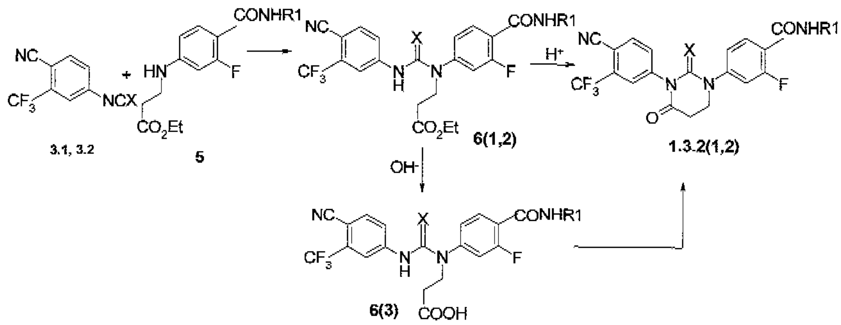


3.1, 3.2 5 6(1,2) 1.3.2(1,2) 6(3)

wherein:

**X** and **R1** have the above meanings.

Scheme 3.

1,4-Diaryl[1,2,4]triazolidin-3,5-diones of the general formula 1.4 are prepared by interaction of the corresponding hydrazine 7 with isocyante **3.1** and subsequent condensation of the prepared semicarbazide 8 with diphosgene according to scheme 4.

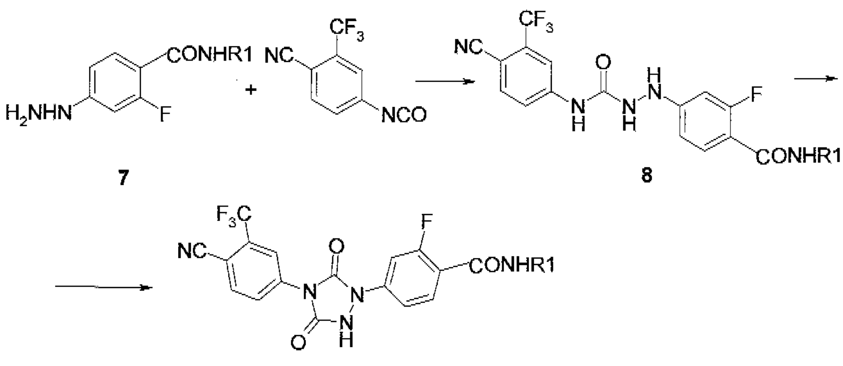

7 8

1.4

wherein:

**R1** has the above meaning.

Scheme 4.

Novel androgen receptor antagonists are also suitable for investigation of molecular mechanism of inhibition and activation of androgen receptors.

Novel cyclic N,N'-diarylthioureas and N,N'-diarylureas of the general formula 1 are androgen receptor antagonists, at that their activity exceeds the activity of known androgen receptor antagonists, published in patent application W02006124118, WO2007127010, and in Drug Data Rep., 2009, 31(6), 609. Besides, novel antagonist **1.2.2(1)** is more than three times as less toxical as **MDV3100** antagonist, because its maximum tolerated dose (MTD), determined in experiments with male mice of CD1 line is equel to MTD > 100 mg/kg, whilst MTD for **MDV3100** is about ~ 30 mg/kg.

The subject of the present invention is novel anticancer agent representing at least one cyclic N,N'-diarylthioureas or N,N'- diarylureas of the general formula **1.**

The subject of the present invention is also novel pharmaceutical composition comprising as an active component at least one cyclic N,N'-diarylthiourea or N,N'-diarylurea of the general formula **1,** its optically active isomer or pharmaceutically acceptable salt exhibiting anticancer activity in effective amount.

The more preferable composition is the pharmaceutical composition exhibiting activity towards prostate cancer comprising as active component at least one cyclic N,N'-diarylthiourea or N,N'-diarylurea of the general formula 1, its optical isomer or pharmaceutically acceptable salt.

Pharmaceutical compositions may include pharmaceutically acceptable excipients. Pharmaceutically acceptable excipients mean diluents, auxiliary agents and/or carriers employing in the sphere of pharmaceutics. According to the invention the pharmaceutical composition in addition to the cyclic N,N'-diarylthiourea or N,N'-diarylurea of the general formula 1, its optically active isomer or pharmaceutically acceptable salt may include other active components, among other things exhibiting anti-cancer activity, provided that they do not give rise to undesirable side-effects.

According to the present invention, if it is necessary to use the pharmaceutical composition in clinical practice it can be mixed up with various traditional pharmaceutical carries.

According to the present invention the carriers used in pharmaceutical compositions represent carriers which are applied in the sphere of pharmaceutics for preparation of commonly used forms including: binding agents, greasing agents, disintegrators, solvents, diluents, stabilizers, suspending agents, colorless agents, taste flavors are used for peroral forms; antiseptic agents, solubilizers, stabilizers are used in the forms for injections; base materials, diluents, greasing agents, antiseptic agents are used in local forms.

The purpose of the present invention is also the method for preparation of pharmaceutical compositions.

The object in view is achieved by mixing novel anti-cancer agent with an inert exicipient and/or solvent, the distinctive feature of which consists in utilization as anticancer agent, at least, one cyclic N,N'-diarylthiourea or N,N'-diarylurea of the general formula 1, its optically active isomer or pharmaceutically acceptable salt. The subject of the present invention is also a medicament in the form of tablets, sheaths or injections including novel anticancer agent or novel pharmaceutical composition intended for cancer treatment.

The preferred medicament including novel anticancer agent or novel pharmaceutical composition is a medicament intended for treatment of prostate cancer.

The subject of the present invention is also therapeutic cocktails for treatment of cancer diseases, among them prostate cancer, incluiding as one of the components novel medicament or novel pharmaceutical composition, comprising as active component at least one cyclic N,N'-diarylthiourea or N,N°-diarylurea of the general formula **1,** its optically active isomer or pharmaceutically acceptable salt.

Therapeutic cocktails for treatment of prostate cancer, along with the medicament disclosed in the invention, may include other known drug substances intended for treatment cancer diseases.

According to the present invention the method for treatment of oncologic diseases, among them, prostate cancer, at humans and warm-blooded animals consists in introduction to human or warm-blooded animal of novel medicament, novel pharmaceutical composition or novel therapeutic cocktail.

Medicaments could be administered perorally or parenterally, for example, intravenously, subcutaneously, intraperitoneally or locally. The clinical dosage of the active component of the general formula 1 could be corrected depending on: therapeutic efficiency and bioavailability of the active ingredients in organism, rate of their exchange and deducing from organism, and also depending on the age, sex and the severity of the patient's symptoms; the daily dosage for adults falls within the range of about 10 to about 500 mg of the active ingredient, preferably of about 50 to about 300 mg. Therefore, according to the present invention in the process of preparation of a medicament from the pharmaceutical composition as units of dosage it is necessary to keep in mind the above effective dosage, so that each unit of dosage should contain of about 10 to about 500 mg of the compound of the general formula **1,** preferably 50 - 300 mg. In accordance with the recommendation of physician or pharmacist the above dosage can be taken several times during the definite time intervals (preferably - from one to six times).

## Best Embodiment of the invention

The invention is illustrated by the following drawings.

**Fig.1.** Weight change of male mice at peroral introduction of compound **1.2.2(1).**
**Fig.2.** Weight change of male mice at peroral introduction of compound **MDV3100.**

The examples given below describe synthesis of N,N'-diarylthioureas and N,N'-diarylureas and data of their biological investigation, which illustrate but not limit the scope of the invention.

## Example 1

Synthesis of *N*-methyl-4-{4-oxo-2-thioxo-3-[3-(trifiuoromethyl)-4-cyanophenyl]imidazolidin-1-yl}-2-fluorobenzamide **1.2(1).** Glycine (80 mg, 1.07 mmol) and $K_2CO_3$ (207 mg, 1.5 mmol) were added to solution of 4-iodo-N-methyl-2-fluorobenzamide (279 mg, 1 mmol) in DMF (3 ml). The reaction mixture was stirred at 140°C for 18 min. in microwave oven, cooled, diluted with AcOEt (10 ml) and water (10 ml), neutralized with HCl to pH 2-3, organic layer was separated, water layer was extracted with AcOEt (5 x 20 ml). The combined extracts were washed with brine, dried over $Na_2SO_4$ and evaporated in *vacuo.* The product was isolated by colomn chromatography on $SiO_2$. It gave N-(4-methylcarbamoyl-2-fluorophenyl)glycine **4.2(1)** (R1=$CH_3$, R4=R5=H,). A solution of *N*-(4-methylcarbamoyl-2-fluorophenyl)glycine **4.2(1)**

(113 mg, 0.5 mmol) and 4-isothiocyanato-2-(trifluoromethyl)benzonirile 3.2 (174 mg, 1.0 mmol) in DMF (2 ml) was stirred at 90°C for 12 h. The reaction mixture was evaporated in *vacuo,* and *N*-methyl-4-{4-oxo-2-thioxo-3-[3-(trifluoromethyl)-4-cyanophenyl]-imidazolidin-1-yl}-2-fluorobenzamide **1.2(1),** was isolated by HPLC method, LCMS $(M+H)^+$ 437.

## Example 2

General method for synthesis of *N*-methyl-4-{5-methyl-4-oxo-2-thioxo-3-[3-(trifluoromethyl)-4-cyanophenyl]imidazolidin-1-yl}-2-fluorobenzamide **1.2(2),** N-methyl-4-{(S)-5-methyl-4-oxo-2-thioxo-3-[3-(trifluoromethyl)-4-cyanophenyl]imidazolidin-1-yl}-2-fluorobenzamide **(S)-1.2(2)** and *N*-methyl-4-{(R)-5-methyl-4-oxo-2-thioxo-3-[3-(trifluoromethyl)-4-cyanophenyl]imidazolidin-1-yl}-2-fluorobenzamide **(R)-1.2(2).**
(D,L)-, (D)- or (L)-Alanine (347 mg, 7.8 mmol) and $Cs_2CO_3$ (2-54 g, 7.8 mmol) were added to the solution of *N*-methyl-2,4-difluorobenzamide (667 mg, 3.9 mmol) in DMSO (3 ml). The reaction mixture was stirred in closed vial at 90°C for 18 h. Cooled mixture was diluted with isopropanol, neutralized with HCl (1.36 ml, 15.6 mmol), filtered, evaporated in *vacuo,* and by HPLC method N-(4-methylcarbamoyl-3-fluorophenyl)alanine **4.2(2)** ($R1=CH_3$, R4=H, $R5=CH_3$), **(S)**-*N*-(4-methylcarbamoyl-3-fluorophenyl)alanine **(S)-4.2(2)** or (R)-*N*-(4-methylcarbamoyl-3-fluorophenyl)alanine **(R)-4.2(2)** were isolated. LCMS $(M+H)^+$ 241. $^1H$ NMR (DMSO-$d_6$, 400 MHz): 12.66 (br. s, 1H), 7.62 (m, 1H), 7.45 (t, *J* = 8.8 Hz, 1H), 6.67 (br. d, *J* = 7.2 Hz, 1 H), 6.42 (dd, $J_1$ = 8.4 Hz, $J_2$ = 2.0 Hz, 1 H), 6.29 (dd, $J_1$ = 14.8 Hz, $J_2$ = 2.0 Hz, 1 H), 4.03 (m, 1 H), 2.73 (d, *J* = 4.4 Hz, 3H), 1.37 (d, *J* = 7.2 Hz, 3H). Solution of amine **4.2(2), (S)-4.2(2)** or **(R)-4.2(2)** (110 mg, 0.46 mmol) and 4-isothiocyanato-2-(trifluoromethyl)-benzonitrile **3.2** (144 mg, 0.55 mmol) in DMF (2 ml) was stirred at 90°C for 12 h in microwave oven, then additional portion of 4-isothiocyanato-2-(trifluoromethyl)-benzonitrile **3.2** (50 mg, 0.19 mmol) was added and stirring was continued for another 12 h. The reaction mixture was evaporated in *vacuo,* and by HPLC method *N*-methyl-4-{5-methyl-4-oxo-2-thioxo-3-[3-(trifluoromethyl)-4-cyanophenyl]imidazolidin-1-yl}-2-fluorobenzamide **1.2(2),** or *N*-methyl-4-{(S)-5-methyl-4-oxo-2-thioxo-3-[3-(trifluoromethyl)-4-cyanophenyl]imidazolidin-1-yl}-2-fluorobenzamide **(S)-1.2(2)** or *N*-methyl-4-{(R)-5-methyl-4-oxo-2-thioxo-3-[3-(trifluoromethyl)-4-cyanophenyl]imidazolidin-1-yl}-2-fluorobenzamide **(R)-1.2(2)** were isolated, respectively. The apparent inhibition constant of androgen receptors ($K_i$) for these compounds are: $K_i^{1.2(2)}$ = 140.2 nM, $K_i^{(S)\text{-}\mathbf{1.2(2)}}$ = 106.7 nM и $K_i^{(R)\text{-}1.2(2)}$ = 73.6 nM, respectively. LCMS $(M+H)^+$ 451. $^1H$ NMR ($CDCl_3$, 400 MHz): 8.28 (t, *J* = 8.6 Hz, 1 H), 8.01 (d, *J* = 8.0 Hz, 1H), 7.94 (d, *J* = 1.2 Hz, 1 H), 7.81 (dd, $J_1$ = 8.0 Hz, $J_2$ = 1.2 Hz, 1 H), 7.48 (dd, $J_1$ = 12.4 Hz, $J_2$ = 1.6 Hz, 1 H), 7.36 (dd, $J_1$ = 8.4 Hz, $J_2$ = 1.6 Hz, 1 H), 6.72 (br. m, 1 H), 4.83 (q, *J* = 7.2 Hz, 1 H), 3.08 (d, *J* = 4.8 Hz, 3H), 1.60 (d, *J* = 7.2 Hz, 3H).

## Example 3

Synthesis of *N*-methyl-4-{2-thio-3-[3-(trifluoromethyl)-4-cyanophenyl]-hydantoin-1-yl}-2-fluorobenzamides **1.2.2** and **1.2.3** (general method). Solution of the corresponding *N*-methyl-2-fluoro-4-[(1-cyanomethyl)amino]benzamide **4.1** (0.75 mmol) and 4-isothiocyanato-2-(trifluoromethyl)benzonitrile **3.2** (342 mg, 1.5 mmol) in DMF (3 ml) was stirred at 110 °C for 12 h in microwave oven. The reaction mixture was dissolved in MeOH (30 ml), 1N HCl (7.5 ml) was added and the resultant mixture was boiled for 1.5 h. The solution was evaporated in *vacuo,* treated with water, the solid was filtered off, washed with water and dried in *vacuo.* The product was isolated by HPLC method. It gave: N-methyl-4-[5-methyl-5-(methoxymethyl)-4-oxo-2-thioxo-3-[3-(trifluoromethyl)-4-cyanophenyl]-imidazolidin-1-yl]-2-fluorobenzamide **1.2.2(1),** $K_i^{1.2.2(1)}$ = 115.9 nM, which was separated to enantiomers by means of high pressure liquid chromatography on Chiralpak HD-H 25x1 cm (Chiral Technologies Inc., USA). Mixture of 80% n-hexane, 20% 2-propanol and 0.02% triethylamine was used as eluent. Flowrate was 4 ml/min. It gave optically pure isomers **(R)-1.2.2(1)** and **(S)-1.2.2(1),** $K_i^{(R)\text{-}1.2.2(1)}$ = 53.3 nM, $K_i^{(S).1.2.2(1)}$ = 721.5 nM. LCMS $(M+H)^+$ 495. $^1H$ NMR ($CDCl_3$, 400 MHz): 8.28 (t, J = 8.4 Hz, 1H), 7.99 (d, *J* = 8.0 Hz, 1 H), 7.92 (s, 1 H), 7.80 (d, *J* = 8.0 Hz, 1 H), 7.29 (dd, $J_1$ = 8.8 Hz, $J_2$ = 1.2 Hz, 1 H), 7.21 (dd, $J_1$ = 11.6 Hz, $J_2$ = 1.2 Hz, 1 H), 6.72 (q, *J* = 4.4 Hz, 1 H), 3.71 (d, *J* = 10.0 Hz, 1 H), 3.43 (s, 3H), 3.35 (d, *J* = 10.0 Hz, 1 H), 3.09 (d, *J* = 4.4 Hz, 3H), 1.52 (s, 3H); *N*-methyl-4-{5-[(benzyloxy)methyl]-5-methyl-4-oxo-2-thioxo-3-[3-(trifluoromethyl)-4-cyanophenyl]imidazolidin-1-yl}-2-fluorobenzamide **1.2.2(2).** LCMS $(M+H)^+$ 571. $^1H$ NMR ($CDCl_3$, 400 MHz): 8.22 (t, *J* = 8.4 Hz, 1 H), 7.96 (d, *J* = 8.0 Hz, 1 H), 7.86 (s, 1 H), 7.70 (dd, $J_1$ = 8.0 Hz, $J_2$ = 1.2 Hz, 1H), 7.39 (m, 3H), 7.29 (m, 2H), 7.25 (dd, $J_1$ = 8.4 Hz, $J_2$ = 1.6 Hz, 1 H), 7.18 (dd, $J_1$ = 8.4 Hz, $J_2$ = 1.6 Hz, 1 H), 6.71 (q, *J* = 4.8 Hz, 1 H), 4.59 (m, 2H), 3.79 (d, *J* = 10.2 Hz, 1 H), 3.45 (d, *J* = 10.2 Hz, 1 H), 3.08 (d, *J* = 4.8 Hz, 3H), 1.51 (s, 3H);
ethyl {4-methyl-3-(4-methylcarbamoyl-3-fluorophenyl)-5-oxo-2-thioxo-1-[3-(trifluoromethyl)-4-cyanophenyl]imidazolidin-4-yl}acetate **1.2.2(4)** ($R1=CH_3$, $R4=CH_3$, $R5=CH_2COOC_2H_5$). LCMS $(M+H)^+$ 536. $^1H$ NMR ($CDCl_3$, 400 MHz): 8.26 (t, *J* = 8.4 Hz, 1H), 8.01 (d, *J* = 8.0 Hz, 1 H), 8.00 (s, 1 H), 7.90 (dd, $J_1$ = 8.0 Hz, $J_2$ = 1.6 Hz, 1 H), 7.18 (dd, $J_1$ = 8.0 Hz, $J_2$ = 1.6 Hz, 1 H), 7.10 (dd, $J_1$ = 8.0 Hz, $J_2$ = 1.6 Hz, 1 H), 6.78 (q, *J* = 4.8 Hz, 1 H), 4.26 (m, 1H), 3.13 (d, *J* = 18.0 Hz, 1 H), 3.09 (d, *J* = *4.8* Hz, 3H), 2.64 (d, *J* = 18.0 Hz, 1 H), 1.67 (s, 3H), 1.31 (t, *J* = 7.0 Hz, 3H); *N*-methyl-4-{4-oxo-2-thioxo-3-[3-(trifluoromethyl)-4-cyanophenyl]-7-oxa-1,3-diazaspiro[4.4]non-1-yl}-2-fluorobenzamide **1.2.3(1),** $K_i^{1.23(1)}$ = 33.9 nM. LCMS $(M+H)^+$ 493. $^1H$ NMR ($CDCl_3$, 400 MHz): 8.30 (t, *J* = 8.4 Hz, 1H), 8.02 (d, *J* = 8.4 Hz, 1 H), 7.98 (d, *J* = 1.6 Hz, 1 H), 7.85 (dd, $J_1$ = 8.4 Hz, $J_2$ = 1.6 Hz, 1 H), 7.34 (dd, $J_1$ = 8.4 Hz, $J_2$ = 1.6 Hz, 1 H), 7.25 (dd, $J_1$ = 11.8 Hz, $J_2$

= 1.6 Hz, 1 H), 6.78 (q, *J* = 4.4 Hz, 1H), 4.43 (d, *J* = 10.0 Hz, 1H), 4.16 (d, *J* = 10.0 Hz, 1 H), 3.96 (m, 1 H), 3.75 (m, 1 H), 3.09 (d, *J* = 4.4 Hz, 3H), 2.74 (m, 1 H), 2.48 (m, 1 H); *N*-methyl-4-{4-oxo-2-thioxo-3-[3-(trifluoromethyl)-4-cyanophenyl]-8-oxa-1,3-diazaspiro[4.5]dec-1-yl}-2-fluorobenzamide **1.2.3(2).** LCMS $(M+H)^+$ 507. $^1$H NMR ($CDCl_3$, 400 MHz): 8.32 (t, *J* = 8.4 Hz, 1 H), 8.01 (d, *J* = 8.0 Hz, 1H), 7.95 (s, 1 H), 7.83 (d, *J* = 8.0 Hz, 1 H), 7.20 (d, *J* = 8.4 Hz, 1H), 7.10 (d, *J* = 8.0 Hz, 1 H), 6.73 (br. m, 1 H), 4.18 (m, 2H), 3.94 (m, 2H), 3.09 (d, *J* = 4.4 Hz, 3H), 2.07 (m, 4H); *N*-methyl-4-{8-methyl-4-oxo-2-thioxo-3-[3-(trifluoromethyl)-4-cyanophenyl]-1,3,8-triazaspiro[4.5]dec-1-yl}-2-fluorobenzamide **1.2.3(3).** $K_i^{\mathbf{1.2.3(3)}}$ = 39.2 nM, $IC_{50}$ =170nM. LCMS $(M+H)^+$ 520. $^1$H NMR (DMSO-$d_6$, 400 MHz): 10.09 (br. s, 1 H), 8.48 (q, *J* = 4.4 Hz, 1 H), 8.43 (d, *J* = 8.4 Hz, 1 H), 8.29 (s, 1 H), 8.11 (d, *J* = 8.4 Hz, 1H), 7.84 (t, *J* = 8.0 Hz, 1H), 7.42 (d, *J* = 10.4 Hz, 1H), 7.30 (d, *J* = 8.0 Hz, 1 H), 3.50 (m, 4H), 2.80 (d, *J* = 4.4 Hz, 3H), 2.78 (s, 3H), 2.72 (d, *J* = 14.0 Hz, 1 H), 2.16 (m, 2H).

**Example 4**

Synthesis of *N*-methyl-[5-(hydroxymethyl)-5-methyl-4-oxo-2-thioxo-3-[3-(trifluoromethyl)-4-cyanophenyl]imidazolidin-1-yl]-2-fluorobenzamide **1.2.2(3).** $BBr_3$ (53 mkl, 0.55 mmol) was added dropwise to solution of *N*-methyl-4-[5-methyl-5-(methoxymethyl)-4-oxo-2-thioxo-3-[3-(trifluoromethyl)-4-cyanophenyl]imidazolidin-1-yl]-2-fluorobenzamide (55 mg, 0.11 mmol) in $CH_2Cl_2$ (1.5 ml) in argon atmosphere at -78°C. The reaction mixture was stirred at -78 °C for 3 h and then for another 3 h at room temperature. After the reaction was completed the excess of $BBr_3$ was neutralized by addition of 5% $Na_2CO_3$ solution (10 ml), the product was extracted with AcOEt, dried over $Na_2SO_4$, evaporated in *vacuo,* and by HPLC method *N*-methyl-4-[5-(hydroxymethyl)-5-methyl-4-oxo-2-thioxo-3-[3-(trifluoromethyl)-4-cyanophenyl]imidazolidin-1-yl]-2-fluorobenzamide **1.2.2(3)** was isolated, $K_i^{1.2.2(3)}$ = 46.3 nM, $^1$H NMR (DMSO-$d_6$, 400 MHz): 8.43 (br. m, 1 H), 8.39 (d, *J* = 8.4 Hz, 1 H), 8.13 (s, 1 H), 7.98 (d, *J* = 8.4 Hz, 1 H), 7.78 (t, *J* = 8.0 Hz, 1 H), 7.42 (d, *J* = 10.8 Hz, 1H), 7.37 (d, *J* = 8.0 Hz, 1N), 5.93 (t, *J* = 4.4 Hz, 1H), 3.81 (dd, $J_1$ = 11.6 Hz, $J_2$ = 4.4 Hz, 1 H), 3.45 (dd, $J_1$ = 11.6 Hz, $J_2$ = 5.0 Hz, 1 H), 2.79 (d, *J* = 4.0 Hz, 3H), 1.38 (s, 3H).

Example 5

Synthesis of {4-methyl-3-(4-methylcarbamoyl-3-fluorophenyl)-5-oxo-2-thioxo-1-[3-(trifluoromethyl)-4-cyanophenyl]imidazolidin-4-yl}acetic acid **1.2.2(5)** ($R1=CH_3$, $R4=CH_3$, $R5=CH_2COOH$). Solution of NaOH (7 mg, 0.172 mmol) in water (0. 5 ml) was added to the solution of ester (46 mg, 0.086 mmol) **1.2.2(4)** in alcohol (2 ml), and the reaction mixture was stirred for 12 h (LCMS control). The solvent was evaporated, isopropanol (2 ml) and HCl (15 mkl, 0.172 mmol) were added, filtered and evaporated again in *vacuo.* {4-Methyl-3-(4-methylcarbamoyl-3-fluorophenyl)-5-oxo-2-thioxo-1-[3-(trifluoromethyl)-4-cyanophenyl]imidazolidin-4-yl}acetic acid **1.2.2(5)** was isolated by HPLC method. LCMS $(M+H)^+$ 469. $^1$H NMR (DMSO-$d_6$, 400 MHz): 13.31 (br. s, 1 H), 8.44 (m, 2H), 8.10 (s, 1 H), 7.95 (d, *J* = 7.6 Hz, 1H), 7.81 (t, *J* = 8.0 Hz, 1 H), 7.25 (d, *J* = 10.8 Hz, 1 H), 7.19 (d, *J* = 8.0 Hz, 1 H), 3.16 (d, *J* = = 17.6 Hz, 1H), 2.79 (d, *J* = 3.6 Hz, 3H), 2.70 (d, *J* = 17.6 Hz, 1 H), 1.59 (s, 3H).

**Example 6**

Synthesis of 4-[3-[3-(trifluoromethyl)-4-cyanophenyl]-2-oxo-tetrahydro-pyrimidin-1(2*H*)-yl]-*N*-methyl-2-fluorobenzamide **1.3.1.** $K_2CO_3$ (109 mg, 0.79 mmol) and 1,3-dibromopropane (32 mkl, 0.32 mmol) were added to a solution of 4-[4-cyano-3-(trifluoromethyl)-phenylcarbamoylamino]-N-methyl-2-fluorobenzamide (100 mg, 0.26 mmol) **2** in DMF (2 ml). Mixture was stirred at 90°C. In 18 h another portion of $K_2CO_3$ (109 mg) and 1,3-dibromopropane (32 mkl) were added and stirring was continued at the same temperature. Addition was repeated by 2 more times. After the reaction was completed the mixture was evaporated in *vacuo,* the residue was dissolved in chloroform, washed with water, dried over $Na_2SO_4$, the solvent was evaporated. The product was isolated by colomn chromatography on $SiO_2$ (eluent -AcOEt). LCMS $(M+H)^+$ 421. $^1$H NMR (DMSO-$d_6$, 400 MHz): 8.17 (br. m, 1H), 8.13 (d, *J* = 8.4 Hz, 1 H), 8.07 (d, *J* = 2.0 Hz, 1H), 7.83 (dd, $J_1$ = 8.4 Hz, $J_2$ = 2.0 Hz, 1H), 7.62 (t, *J* = 8.4 Hz, 1 H), 7.38 (dd, $J_1$ = 12.4 Hz, $J_2$ = 2.0 Hz, 1 H), 7.29 (dd, $J_1$ = 8.4 Hz, $J_2$ = 2.0 Hz, 1 H), 3.90 (t, *J* = 5.8 Hz, 2H), 3.81 (t, *J* = 5.8 Hz, 2H), 2.77 (d, *J* = 4.8 Hz, 3H), 2.21 (m, 2H).

**Example 7**

Synthesis of *N*-methyl-4-{[3-(trifluoromethyl)-4-cyanophenyl]-2,4-dioxo-tetrahydropyrimidin-1(2*H*)-yl}benzamide **1.3.2(1),** ($X=O$, $R1=CH_3$). Ethyl acrylate (8 g, 80 mmol) and DBU (0.81 g, 5.4 mmol) were added to a solution of 4-amino-*N*-methyl-2-fluorobenzamide (9 g, 53.6 mmol) in DMSO (90 ml) and stirring was continued for 24 h at 70°C (LCMS control). The reaction mixture was subjected to lyophilization, the residue was recrystallized from aqueous alcohol. It gave ethyl N-[4-(methylcarbamoyl)-3-fluorophenyl]-β-alaninate **5.** LCMS $(M+H)^+$ 269. $^1$H NMR (DMSO-$d_6$, 400 MHz) $\delta$ 7.57 (br. s, 1 H), 7.48 (t, *J* = 8.8 Hz, 1 H), 6.47 (br. s, 1H), 6.42 (d, *J* = 8.8 Hz, 1 H), 6.33 (d, *J* = 14.8 Hz, 1 H), 4.07 (q, *J* = 7.2 Hz, 2H), 3.32 (br. m, 2H), 2.73 (d, *J* = 4.4 Hz, 3H), 2.55 (t, *J* = 6.4 Hz, 2H), 1.18 (t, *J* = 7.2 Hz, 3H).

The solution of 4-isocyanato-2-(trifluoromethyl)benzonitrile (425 mg, 1.87 mmol) **3.1** and ethyl *N*-[4-(methylcarbamoyl)-3-fluorophenyl]-β-alaninate (500 mg, 1.87 mmol) 5 (R1=$CH_3$) in $CH_2Cl_2$ (10 ml) was stirred for 5 h. The reaction mixture was evaporated in *vacuo,* and the product was isolated by colomn chromatography on $SiO_2$ (eluent - n-hexane : AcOEt : $Et_3N$ = 1:1:0.03). It gave ethyl *N*-[4-(methylcabamoyl)-3-fluorophenyl]-*N*-{[3-(trifluoromethyl)-4-cyanophenyl]-carbamoyl}-β-alaninate **6(1)** (R1=$CH_3$, X=O). LCMS $(M+H)^+$ 481. HCl (2.5 ml) was added to the solution of ethyl *N*-[4-(methylcabamoyl)-3-fluorophenyl]-*N*-{[3-(trifluoromethyl)-4-cyanophenyl]-carbamoyl}-β-alaninate (500 mg, 1.04 mmol) **6(1)** in AcOH (5 ml) and the resultant mixture was stirred for 15 h. The reaction mixture was poured into water, the product was extracted with EtOAc. Organic layer was dried over $Na_2SO_4$, evaporated in *vacuo* and by means of colomn chromatography on $SiO_2$ (eluent - n-hexane : AcOEt = 1:1) *N*-methyl-4-{[3-(trifluoromethyl)-4-cyanophenyl]-2,4-dioxo-tetrahydropyrimidin-1(2*H*)-yl}benzamide **1.3.2,** (X=O, R1=$CH_3$) was isolated; $K_i^{1.3.2(1)}$ = 85.6 nM, LCMS $(M+H)^+$ 435. $^1H$ NMR (DMSO-$d_6$, 400 MHz): δ 8.29 (d, *J* = 7.6 Hz, 1H), 8.23 (br. m, 1H), 8.11 (s, 1 H), 7.89 (d, *J* = 2.0 Hz, 1 H), 7.67 (t, *J* = 8.4 Hz, 1 H), 7.39 (d, *J* = 12.4 Hz, 1H), 7.33 (d, *J* = 8.4 Hz, 1 H), 4.02 (t, *J* = 6.4 Hz, 2H), 3.03 (t, *J* = 6.4 Hz, 2H), 2.77 (d, *J* = 4.4 Hz, 3H).

**Example 8**

Synthesis of *N*-methyl-4-{[3-(trifluoromethyl)-4-cyanophenyl]-4-oxo-2-thioxo-tetrahydropyrimidin-1(2*H*)-yl}benzamide **1.3.2(2),** (X=S, R1=$CH_3$). A solution of 4-isothiocyanato-2-(trifluoromethyl)benzonitrile (320 mg, 1.51 mmol) **3.2** and ethyl *N*-[4-(methylcarbamoyl)-3-fluorophenyl]-β-alaninate (404 mg, 1.51 mmol) **5** (R1=$CH_3$) in DMF (8 ml) was heated at 60°C in microwave stove for 8 h. The reaction mixture was evaporated in *vacuo,* and by means of colomn chromatography on $SiO_2$ (eluent - n-hexane : AcOEt = 1:2) ethyl *N*-[4-(methylcarbamoyl)-3-fluorophenyl]-*N*-{[3-(trifluoromethyl)-4-cyanophenyl]thiocarbamoyl}-β-alaninate **6(2)** (R1=$CH_3$, X=S) was isolated. LCMS $(M+H)^+$ 497. A solution of NaOH (32 mg, 0.8 mmol) in water (0.25 ml) was added to a solution of ester (200 mg, 0.4 mmol) **6(2)** in alcohol (1 ml), and the resultant mixture was stirred at 80°C for 2 h (LCMS control), cooled and neutralized with HCl (69 mkl, 0.8 mmol), evaporated in *vacuo,* the residue was extracted with hot isopropanol and evaporated in *vacuo* again. It gave *N*-[4-(methylcarbamoyl)-3-fluorophenyl]-*N*-{[3-(trifluoromethyl)-4-cyanophenyl]thiocarbamoyl}-β-alanine **6(3)** (R1=$CH_3$, X=S). LCMS $(M+H)^+$ 469. TBTU (86 mg, 0.36 mmol) and diisopropylethylamine (110 mg, 0.84 mmol) were added to the solution of the prepared acid (114 mg, 0.24 mmol) **6(3)** in DMF (1.5 ml). The reaction mixture was stirred at 45°C for 15 h. When the reaction was completed (LCMS control) the solution was poured into water and extracted with EtOAc. The organic layer was dried over $Na_2SO_4$, evaporated in *vacuo* and by HPLC method *N*-methyl-4-{[3-(trifluoromethyl)-4-cyanophenyl]-4-oxo-2-thioxo-tetrahydropyrimidin-1(2*H*)-yl}-2-fluorobenzamide **1.3.2(2)** (R1=$CH_3$, X=S) was isolated; $K_i^{\mathbf{1.3.2(2)}}$ = 95.2 nM. LCMS $(M+H)^+$ 451. $^1H$ NMR (DMSO-$d_6$, 400 MHz): 8.35 (q, *J* = 4.4 Hz, 1 H), 8.27 (d, *J* = 8.0 Hz, 1 H), 8.06 (d, *J* = 1.6 Hz, 1 H), 7.83 (dd, $J_1$ = 8.0 Hz, $J_2$ = *1.6* Hz, 1 H), 7.71 (t, *J* = 8.2 Hz, 1 H), 7.42 (dd, $J_1$ = 11.0 Hz, $J_2$ = 1.8 Hz, 1 H), 7.33 (dd, $J_1$ = 8.2 Hz, $J_2$ = 1.8 Hz, 1 H), 4.13 (t, *J* = 6.8 Hz, 2H), 3.17 (t, *J* = 6.8 Hz, 2H), 2.78 (d, *J* = 4.4 Hz, 3H).

**Example 9**

Synthesis of *N*-methyl-2-fluoro-4-[4-[3-(trifluoromethyl)-4-cyanophenyl]-3,5-dioxo-1,2,4-triazolidin-1-yl]benzamide **1.4** (R1=$CH_3$). 2.5M Solution of $NaNO_2$ (2.38 ml) was added dropwise to a solution of 4-amino-N-methyl-2-fluorobenzamide (1 g, 5.95 mmol) in B 5N HCl (3.1 ml), so that the temperature of the reaction mixture did not exceed 5°C. The mixture was stirred for additional 30 min at the same temperature, after that the prepared solution was added drop by drop to a suspension of $SnCl_2$*$2H_2O$ (4.03 g, 17.9 mmol) in HCl (4.2 ml) at 0°C, and stirring was continued for 2 h at the same temperature. Precipitated solid was filtered off, dissolved in water (40 ml) and NaOH was added to strongly basic reaction. The mixture was extracted with ether (3*100 ml), dried over $MgSO_4$ and evaporated in *vacuo.* It gave 4-hydrazino-*N*-methyl-2-fluorobenzamide **7** (R1=$CH_3$). LCMS $(M+H)^+$ 184. $^1H$ NMR ($CDCl_3$, 400 MHz): 7.96 (t, *J* = 8.4 Hz, 1 H), 6.64 (br. m, 1 H), 6.60 (t, *J* = 1.6 Hz, 1 H), 6.57 (dd, $J_1$ = 7.2 Hz, $J_2$ = 2.0 Hz, 1 H), 5.60 (br. s, 1 H), 3.66 (br. s, 2H), 3.00 (dd, $J_1$ = 4.8 Hz, $J_2$ = 1.2 Hz, 1 H). The solution of 4-isocyanato-2-(trifluoromethyl)benzonitrile (59 mg, 0.27 mmol) **3.1** in dioxane (2 ml) was added to a solution of 4-hydrazino-N-methyl-2-fluorobenzamide (54 mg, 0.29 mmol) 7 in dioxane (3 ml), and the resultant mixture was stirred for 2 h. Then dioxane was distilled in *vacuo,* the residue was crumbled with ether, filtered off and dried in *vacuo.* It gave 2-[(4-methylcarbamoyl)-3-fluorophenyl]-*N*-[3-(trifluoromethyl)-4-cyanophenyl]-hydrazine carboxamide 8(1) (R1=$CH_3$). LCMS $(M+H)^+$ 405. $^1H$ NMR (DMSO-$d_6$, 400 MHz): 9.65 (br. s, 1 H), 8.72 (br. s, 1 H), 8.37 (s, 1H), 8.25 (br. s, 1 H), 8.03 (br. m, 1 H), 7.88 (d, *J* = 8.8 Hz, 1H), 7.58 (m, 2H), 6.63 (d, *J* = 8.4 Hz, 1 H), 6.48 (d, *J* = 14.0 Hz, 1H), 2.77 (d, *J* = 4.4 Hz, 3H). Triethylamine (56 mkl, 0.4 mmol) and diphosgene (27 mkl, 0.22 mmol) were added one after another to 2-[(4-methylcarbamoyl)-3-fluorophenyl]-*N*-[3-(trifluoromethyl)-4-cyanophenyl]-hydrazine carboxamide (80 mg, 0.2 mmol) **8(1)** in dichloroethane (2 ml). The reaction mixture was stirred in a closed vial at 80 °C for 15 h. The solvent was evaporated in vacuo and the residue was subjected to chromatography on $SiO_2$ (eluent - $CH_2Cl_2$: MeOH, gradient from 100:1 till 20:1). It gave *N*-methyl-2-fluoro-4-[4-[3-(trifluoromethyl)-4-cyanophenyl]-3,5-dioxo-1,2,4-triazolidin-1-yl]benzamide **1.4** (R1=$CH_3$). $K_i^{1.4}$ = 55.2 nM, LCMS $(M+H)^+$ 422. $^1H$ NMR (DMSO-$d_6$, 400 MHz): 11.53 (s, 1 H), 8.22 (br. m, 1 H), 8.16 (d, $J$ = 8.8 Hz, 1 H), 7.99 (dd, $J_1$ = 8.8 Hz, $J_2$ = 1.6 Hz, 1 H), 7.95 (d, $J$ = 1.6 Hz, 1 H), 7.81 (t, $J$ = 8.4 Hz, 1 H), 7.69 (dd, $J_1$ = 8.8 Hz, $J_2$ = 2.0 Hz, 1 H), 7.62 (dd, $J_1$ = 12.0 Hz, $J_2$ = 2.0 Hz, 1 H), 2.79 (d, $J$ = 4.4 Hz, 3H).

**Example 10**

Determination of antagonistic activity of cyclic N,N'-diarylthioureas and N,N'-diarylureas of the general formula 1 and their analog **MDV3100** towards androgen receptors. The ability of novel cyclic N,N'-diarylthioureas and N,N'-diarylureas of the general formula 1 and **MDV3100** agent to block androgen receptors was determined by their effectiveness of inhibition of dihydrotestosterone stimulated expression of prostate specific antigen (PSA) in cancer cells of human prostrate LNCap, derived from the American Tissue Culture Collection (ATCC, USA). These cells are sensitive towards 5-$\alpha$-dihydrotestosterone (DHT) and in its presence produce cancer markers (PSA). The cells were cultured in RPMI 1640 medium (Invitrogen, USA) containing 10% calf serum (Hyclone, USA), 1% antibacterial/antifungal mixture (Sigma, USA) and 4,5% glucose. Before the experiment the cells were washed and suspended in the same medium in which, however, instead of calf serum the serum which had been treated with charcoal for removal of hormone traces was used. The cells were embedded into wells of 96-well plates by 100 $\mu$l per cell (10 000 cells) and left for 4 days in incubator at 37°C (100% humidity) in atmosphere of 95% air/5% $CO_2$. After incubation cyclic N,N'-diarylthioureas or N, N'-diarylureas of the general formula 1 were added to the cells in various concentrations, and then - 20nM DHT (concentration corresponding to 80-90% of maximum stimulation). The cells were left for 5 days for additional incubation under the same conditions. After that the samples of supracellular medium were taken on analysis for PSA content. The test was carried out according to the protocol, recommended by manufacturer of the kit for determination of PSA (Alpha Diagnostic International, USA). After wetting the wells containing PSA antibodies attached to their bottom to each well 25 $\mu$l of the tested compounds and 100 $\mu$l of PSA antibodies conjugated previously with horseradish peroxidase were added successively.

After incubation at room temperature for 30 minutes, the contents of the wells were removed, the wells were washed several times, and then 100 $\mu$l of chromogenic substrate of peroxidase was added to each well. Plates were held for 15 min. at room temperature, after that 50 $\mu$l of stop solution was added to every well; at that a dye is formed the absorption intensity of which was mesured at 450 nM; the value obtained is proportional to PSA concentration in the sample. Based on the dependence of lowering of PSA synthesis, caused by dihydrotestosterone (DHT), on the concentration of the tested compounds, dose-response curves were plotted, from which $IC_{50}$ values were determined. They were used for calculation of the values of apparent inhibition constants ($K_i$) for the compounds of the general formula I according to Cheng-Prusoff equation. [Cheng, Y., Prusoff, W. H. "Relationship between the inhibition constant (Ki) and the concentration of inhibitor which causes 50 per cent inhibition (IC50) of an enzymatic reaction". Biochem Pharmacol. (1973) 22, 3099-3108]:

$$K_i = IC_{50}/(1+L/K_D),$$

wherein L - agonist concentration (DHT), $K_D$ - receptor activation constant, numerically equal to $EC_{50}$ value, which is determined in every experiment according to dependence of stimulation of PSA synthesis on DHT concentration. The data obtained given in the corresponding examples testify that novel androgen receptor antagonists, in some cases, are more active than MDV3100, tested under the same conditions as a compound for comparison, for which $K_i^{MDV3100}$ = 79.5 nM.

**Example 11**

Determination of maximum tolerated dose of novel antagonists **1.2.2(1),** and 1.2.3(3) and its analog **MDV3100.** Maximum tolerated dose (MTD) of novel antagonists **1.2.2(1)** and **1.2.3(3)** and its analog **MDV3100** were determined in experiments on male mice of CD1 line at peroral administration 1 time a day within 5 days in doses 10, 30 and 100 mg/kg. The compound was dissolved in sterile water with addition of Twin-80. Sterile water with Twin-80 was introduced to control animals (Placebo group). Body weight was appreciated, and also animals' mortality rate. Statistical comparison of groups was carried out according to non-parametric test ANOVA, with the use of Statistica programme.

At the introduction of compounds in dose up to 100 mg/kg mice death was not observed. On the 3rd - 4th day body weight of mice in the group, received the tested compound in dose 100 mg/kg was less in comparison with the body weight of control animals, however, statistical significance at this was not observed (fig.1). The data show that compound has MTD > 100 mg/kg.

At the introduction of **MDV3100** in doses 10 and 30 mg/kg mice death was not observed. In the group of mice to which the tested compound was introduced in dose 100 mg/kg, the body weight began to lower on the $3^{rd}$ day. On the $5^{th}$ day body weight of this group of animals statistically differed from body weight of animals from Placebo group (p=0,002, fig. 2). One animal died. The data show that compound **MDV3100** has MTD ~ 30 mg/kg.).

**Example 12**

Preparation of medicament in the form of tablets. Starch (1600 mg), grained lactose (1600 mg), talcum (400 mg) and *N*-methyl-4-[5-methyl-5-(methoxymethyl)-4-oxo-2-thioxo-3-[3-(trifluoromethyl)-4-cyanophenyl]-imidazolidin-1-yl]-2-fluorobenzamide **(R)-1.2.2(1)** (1000 mg) mixed together and pressed in a brick. Prepared brick was crushed to granules and riddled through sieves, gathering granules of 14-16 mesh size. The obtained granules were pelletised in tablets of suitable form of 560 mg by weight each.

**Example 13**

Preparation of medicament in the form of capsules. N-Methyl-4-[5-methyl-5-(methoxymethyl)-4-oxo-2-thioxo-3-[3-(trifluoromethyl)-4-cyanophenyl]-imidazolidin-1-yl]-2-fluorobenzamide **(R)-1.2.2(1)** was carefully mixed with lactose powder in ratio 2 : 1. The prepared pharmaceutical composition was packed on 300 mg into gelatinous capsules of suitable size.

**Example 16**

Preparation of medicament in the form of compositions for intramuscular, intraperitoneal or hypodermic injections. N-Methyl-4-[5-methyl-5-(methoxymethyl)-4-oxo-2-thioxo-3-[3-(trifluoromethyl)-4-cyanophenyl]-imidazolidin-1-yl]-2-fluorobenzamide **(R)-1.2.2(1)** (500 mg) was dissolved in the mixture of chlorobutanole (300 mg), propylene glycol (2 ml), and water for injections (100 ml). The prepared solution was filtered and placed in 1 ml ampoules which were sealed up and sterilized in an autoclave.

**Industrial applicability**

The present invention could be used in medicine, veterinary, biochemistry.

## Claims

1. A cyclic N,N'-diarylthioureas and *N,N'*-diarylurea compound of general formula 1, or optical (R)- or (S)- isomer, or a pharmaceutically acceptable salt thereof, exhibiting properties of androgen receptor antagonists,

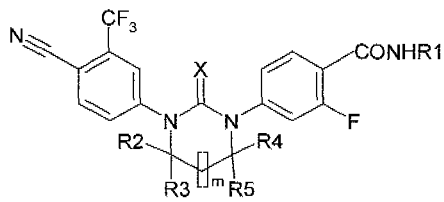

**1**

wherein:

**X** represents oxygen or sulfur;
**m** = 0 or 1;
**R1** represents $C_1$-$C_3$alkyl;
**R2** and **R3** represent hydrogen; or
**R2** and **R3** together with the carbon atom they are attached to form C = O group;
**R4** and **R5** represent hydrogen; or
**R4** represents hydrogen, **R5** represents methyl; or
**R4** represents methyl, **R5** represents **$CH_2$R6** group in which **R6** is $C_1$-$C_3$ alkoxycarbonyl, carboxyl, hydroxyl

group optionally substituted with methyl or benzyl; or

**R4** and **R5** together with the carbon atom they are attached to form 5- or 6-membered saturated heterocycle including at least one oxygen atom or nitrogen atom optionally substituted with methyl, or

**R4** and **R5** together with the carbon atom they are attached to represent NH-group.

**2.** The compound of claim 1 selected from cyclic *N,N'*-diarylthioureas and *N,N'*-diarylureas of general formulas **1.2, 1.3** or **1.4,** or optical (R)- and (S)- isomers, or a pharmaceutically acceptable salt thereof,

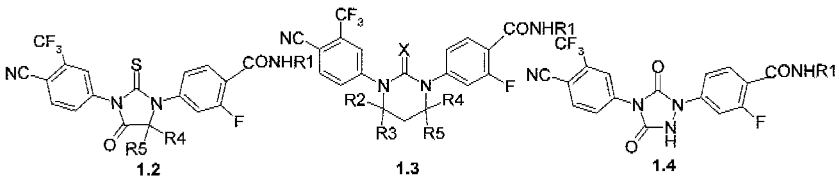

1.2 1.3 1.4

wherein:

**X, R1** , **R2, R3,** R4 and **R5** are all as mentioned above.

**3.** The compound of claim 2 selected from the group consisting of cyclic N,N'-diarylthioureas of formulas **1.2(1), 1.2 (2), 1.2.2,** and **1.2.3,** or optical (R)-isomers - (R)-**1.2(2)**, (R)-**1.2.2,** (R)-**1.2.3,** or (S)-isomers - (S)-**1.2(2)**, (S)-**1.2.2** and (S)-**1.2.3,** or a pharmaceutically acceptable salt thereof,

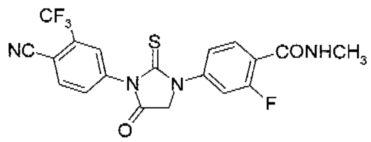

**1.2(1)**

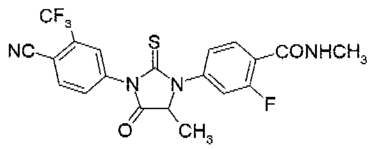

**1.2(2)**

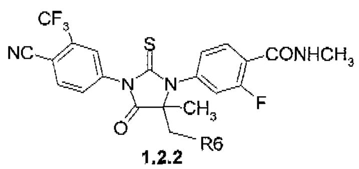

**1.2.2**

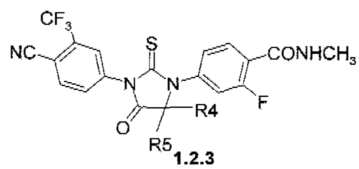

**1.2.3**

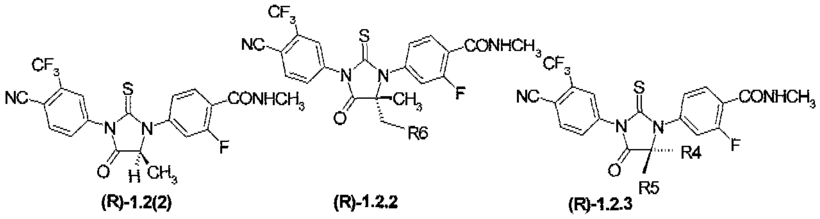

**(R)-1.2(2)** **(R)-1.2.2** **(R)-1.2.3**

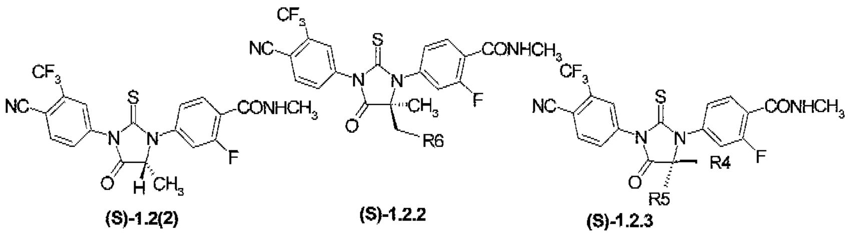

(S)-1.2(2) (S)-1.2.2 (S)-1.2.3

wherein:

**R4** and **R5** together with the carbon atom they are attached to form 5- or 6-membered saturated heterocycle comprising at least one oxygen atom or nitrogen atom optionally substituted with methyl,
**R6** has the above meanings.

4. The compound of claim 2 selected from the group consisting of compounds of formulas **1.2.2(1), 1.2.2(2), 1.2.2(3), 1.2.3(1), 1.2.3(2)** and **1.2.3(3),** or optical (R)-isomers - (R)-**1.2.2(1),** (R)**-1.2.2(2),** (R)-**1.2.2(3),** (R)-**1.2.3(1),** or (S)-isomers - (S)-**1.2.2(1),** (S)-**1.2.2(2),** (S)-**1.2.2(3),** (S)**-1.2.3(1),** or a pharmaceutically acceptable salt thereof,

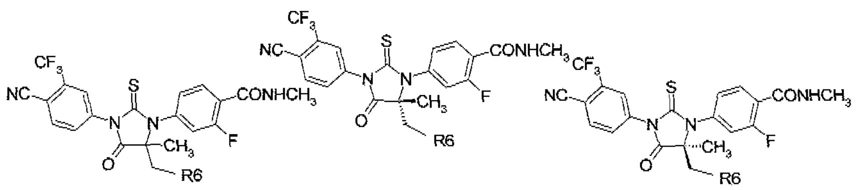

**1.2.2(1)**: R6 =$OCH_3$; **1.2.2(2)**: R6 =$OCH_2Ph$; **1.2.2(3)**: R6 = OH;

**(R)-1.2.2(1)**: R6 = $OCH_3$; **(R)-1.2.2(2)**: R6 =$OCH_2Ph$; **(R)-1.2.2(3)**: R6 = OH;

**(S)-1.2.2(1)**: R6 =$OCH_3$; **(S)-1.2.2(2)**: R6 = $OCH_2Ph$; **(S)-1.2.2(3)**: R6 = OH;

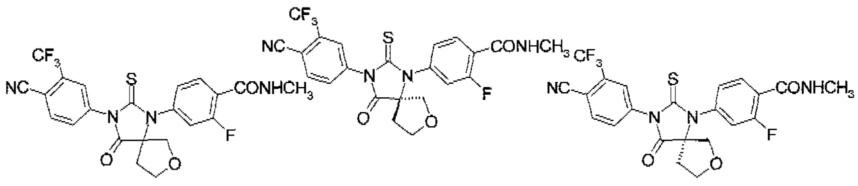

**1.2.3(1)** **(R)-1.2.3(1)** **(S)-1.2.3(1)**

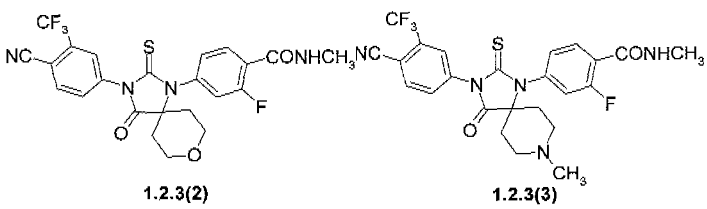

**1.2.3(2)** **1.2.3(3)**

wherein:

**R6** is hydroxyl group optionally substituted with methyl or benzyl.

5. Method for preparation of compounds of the general formula **1.2** and their optical **(R)-** and **(S)-** isomers according to claims 1-4 by interaction of the corresponding 4-(cyanomethyl)amino-benzamides **4.1** or (4-carbamoylphenylamino)-acetic acids **4.2** or their optical **(R)-** and **(S)-**isomers with isothiocyanate **3.2.**

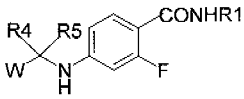

**4.1**: W = CN
**4.2**: W = $CO_2H$

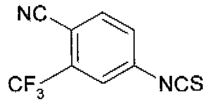

**3.2**

6. Anticancer agent representing at least one cyclic N,N'-diarylthiourea or N,N'-diarylurea of the general formula 1 according to any of claims 1 - 4 exhibiting properties of androgen receptor antagonists.

7. Pharmaceutical composition exhibiting properties of androgen receptor antagonist comprising as active component anticancer agent according to claim 6.

8. Method for preparation of pharmaceutical composition according to claim 7 by mixing anticancer agent according to claim 6 with inert filler and/or solvent.

9. A medicament in the form of tablets, sheaths or injections for treatment of cancerous diseases comprising anticancer agent according to claim 6 or pharmaceutical composition according to claim 7.

10. The medicament according to claim 9 intended for prostate cancer treatment.

11. A method for treatment of cancerous diseases, among them prostate cancer, by introduction of medicament according to any of claims 9, 10, or pharmaceutical composition according to claim 7, or anticancer agent according to claim 6.

12. Androgen receptor antagonists according to claim 1 for investigation of molecular mechanism of inhibition and activation of androgen receptors.

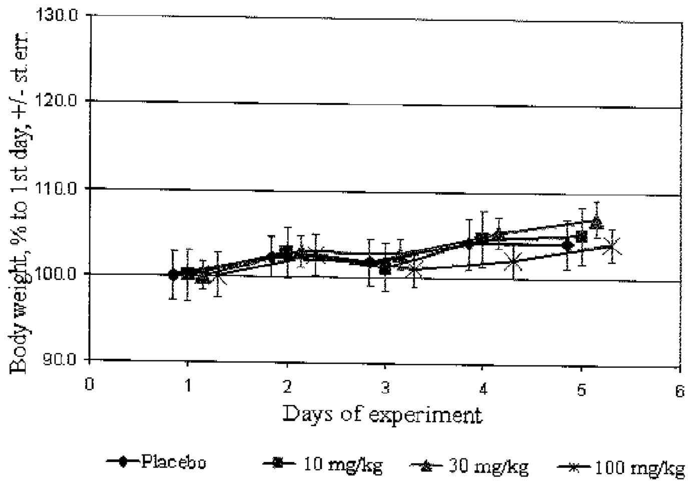
130.0
120.0
110.0
100.0
90.0
Body weight, % to 1st day, +/- st.err.
0
1
2
3
4
5
6
Days of experiment
Placebo
10 mg/kg
30 mg/kg
100 mg/kg

Fig. 1.

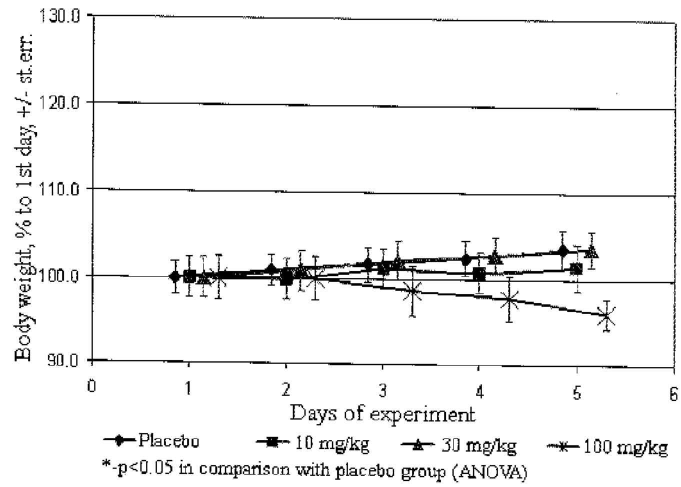
130.0
120.0
110.0
100.0
90.0
Body weight, % to 1st day, +/- st.err.
0
1
2
3
4
5
6
Days of experiment
Placebo
10 mg/kg
30 mg/kg
100 mg/kg
*-p<0.05 in comparison with placebo group (ANOVA)

Fig. 2.

**INTERNATIONAL SEARCH REPORT**

International application No.
PCT/RU 2011/000476

A. CLASSIFICATION OF SUBJECT MATTER

see supplemental sheet

According to International Patent Classification (IPC) or to both national classification and IPC

B. FIELDS SEARCHED

Minimum documentation searched (classification system followed by classification symbols)

C07C 275/32, C07D 471/10, 233/86, 233/30, 233/72, 249/12, 239/10, 239/22, 249/10, 491/107, A61K 31/4166, 31/4188, 31/513, 31/4196, 31/527, 9/20, 9/48, 9/08, A61P 35/00

Documentation searched other than minimum documentation to the extent that such documents are included in the fields searched

Electronic data base consulted during the international search (name of data base and, where practicable, search terms used)

STN, RUPAT, EAPO, Esp@cenet, PAJ, USPTO, CIPO, DEPATIS, PCT Online

C. DOCUMENTS CONSIDERED TO BE RELEVANT

| Category* | Citation of document, with indication, where appropriate, of the relevant passages | Relevant to claim No. |
|---|---|---|
| X | WO 2006/124118 A1 (THE REGENTS OF THE UNIVERSITY OF CALIFORNIA) 23.11.2006, p.p. 1, 3, 5-7, 9-11, 13, 20-23, 25-40, 45, 46 the claims, p. 130, 131, paragraphs [0030], [0162], examples | 1-12 |
| X | WO 2007/127010 A2 (THE REGENTS OF THE UNIVERSITY OF CALIFORNIA) 08.11.2007, p.p. 1, 2, 5, 7, 10-22, 25-40 the claims, paragraphs [00161], [0030], [0162], example, tables 2-9 | 1-12 |
| X | PRAKASH VISHNU et al. Update on options for treatment of metastatic castration-resistant prostate cancer. OncoTargets and Therapy, 2010, 3, 39-51, (the abstract), p. 48, column 1, section MDV3100, p. 49, table 3, column 2, line 2 | 1-12 |
| X | TRAN CHRIS et al. "Development of a Second-Generation Antiandrogen for Treatment of Advanced Prostate Cancer". Science, 2009, 324 (5298), pp. 787-790 (the abstract) [on-line] Found from ACS on STN, CA: 151: 115902 | 1-12 |
| A | RU 2152934 C1 (RUSSEL YUKLAF) 20.07.2000 | 1-12 |

☐ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 15 November 2011 (15.11.2011) | 08 December 2011 (08.12.2011) |
| Name and mailing address of the ISA/ | Authorized officer |
| Facsimile No. | Telephone No. |

Form PCT/ISA/210 (second sheet) (July 1998)

INTERNATIONAL SEARCH REPORT

International application No.
PCT/RU 2011/000476

***C07D 239/10*** *(2006.01)*
***C07D 239/22*** *(2006.01)*
***C07D 233/86*** *(2006.01)*
***C07D 233/72*** *(2006.01)*
***C07D 471/10*** *(2006.01)*
***C07D 491/107*** *(2006.01)*
***C07D 249/12*** *(2006.01)*
***A61K 31/4196*** *(2006.01)*
***A61K 31/513*** *(2006.01)*
***A61K 31/4166*** *(2006.01)*
***A61K 31/4188*** *(2006.01)*
***A61K 31/527*** *(2006.01)*
***A61K 9/20*** *(2006.01)*
***A61K 9/48*** *(2006.01)*
***A61K 9/08*** *(2006.01)*
***A61P 35/00*** *(2006.01)*

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006124118 A
- WO 2007127010 A


### Non-patent literature cited in the description


- *Drug Data Rep.,* 2009, vol. 31 (6), 609
- **BERGE S.M. et al.** Pharmaceutical Salts. *J.Pharm.Sci.,* 1977, vol. 66, 1-19
- **CHENG, Y. ; PRUSOFF, W. H.** Relationship between the inhibition constant (Ki) and the concentration of inhibitor which causes 50 per cent inhibition (IC50) of an enzymatic reaction. *Biochem Pharmacol.,* 1973, vol. 22, 3099-3108